# EUROPEAN PATENT SPECIFICATION

(11) **EP 2 889 045 B1**
(45) Date of publication and mention of the grant of the patent: **27.09.2017**
(21) Application number: 14773062.6
(22) Date of filing: 25.03.2014
(51) Int. Cl.: A61L 2/22, A61L 2/18, A61L 2/24, B01L 1/00, B05B 1/30, B05B 7/04, C12M 1/00

(54) **ISOLATOR SYSTEM**
ISOLATORSYSTEM
SYSTÈME D'ISOLATION

(30) Priority: 29.03.2013 JP 2013071716; 29.03.2013 JP 2013071697
(43) Date of publication of application: 01.07.2015
(73) Proprietor: Panasonic Healthcare Holdings Co., Ltd., Tokyo 105-8433 (JP)
(72) Inventor: KOBAYASHI, Koichi, Toon-shi, Ehime 791-0395 (JP); YOKOI, Yasuhiko, Toon-shi, Ehime 791-0395 (JP); SEKINE, Hironobu, Toon-shi, Ehime 791-0395 (JP); OGUMA, Ritarou, Toon-shi, Ehime 791-0395 (JP)
(74) Representative: Grünecker Patent- und Rechtsanwälte PartG mbB
(86) International application number: PCT/JP2014/001727
(87) International publication number: WO 2014/156139

(56) References cited:
- EP-A1- 2 210 618
- EP-A1- 2 517 734
- EP-A1- 2 692 848
- WO-A1-2012/132146
- JP-A- 2000 288 075
- JP-A- 2004 524 086
- JP-A- 2006 320 392
- JP-A- 2008 011 928
- JP-A- 2010 063 967
- JP-A- 2012 231 917
- JP-U- H02 133 432
- US-A1- 2010 189 607

## Description

### [Technical Field]

The present disclosure relates to an isolator used in laboratory environment equipment related to regenerative medicine and pharmaceutical production according to the preamble of claim 1.

### [Background]

An isolator as described in the preamble of claim 1 is already known from US 2010/189607 A1. Similar isolators are disclosed in EP 2692848 A1, EP 2 210 618 A1 and EP 2 517 734 A1. EP 2 692 848 A1 is an intermediate document. Patent Literature 1 discloses an isolator used in cell manipulation, culture works, and the like. A sterilizing substance supply device used in this isolator includes a sterilizing gas generation unit constituted by an atomizing unit and an evaporating unit. A hydrogen peroxide solution that is converted into mist in the atomizing portion is heated by a heater to be evaporated in the evaporating portion. The evaporated hydrogen peroxide solution is supplied as a sterilizing gas to the working chamber of the isolator.

### [Citation List]

### [Patent Literature]

Japanese Patent Application Laid-Open Publication No. 2010-194301

### [Summary]

### [Technical Problem]

Hence, the present disclosure is to provide an isolator configured to reduce a period of time required for sterilizing processing.

### [Solution to Problem]

The above and other objects of the invention are solved by the isolator according to claim 1. Preferred embodiments are claimed in the dependent claims.

### [Advantageous Effects]

The isolator in the present disclosure is effective in reducing a period of time required for sterilizing processing.

### [Brief Description of the Drawings]

Fig. 1 is an exemplary front view illustrating an isolator system according to a first embodiment.
Fig. 2 is an exemplary perspective view illustrating the isolator system according to the first embodiment.
Fig. 3 is an exemplary perspective view illustrating the isolator system when an incubator is mounted according to the first embodiment.
Fig. 4 is an exemplary schematic view illustrating a configuration of the isolator system according to the first embodiment.
Fig. 5 is an exemplary partial perspective view illustrating an internal configuration of the isolator system according to the first embodiment.
Fig. 6 is an exemplary schematic view illustrating a configuration of a nozzle according to the first embodiment.
Fig. 7 is an exemplary partial front view illustrating an internal configuration of the isolator system according to the first embodiment.
Fig. 8 is an exemplary cross-sectional view taken along line a-a in Fig. 7.
Fig. 9 is an exemplary cross-sectional view taken along line b-b in Fig. 7.
Fig. 10 is an exemplary block diagram illustrating an example of control of a control unit according to the first embodiment.
Fig. 11 is an exemplary timing chart illustrating an example of control of the control unit according to the first embodiment.
Fig. 12 is an exemplary timing chart illustrating an example of control of the control unit according to another embodiment.

### [Mode for Carrying Out the Disclosure]

Hereinafter, embodiments will be described in detail with reference to drawings as necessary. However, more detailed description than necessity may be omitted. For example, the detailed descriptions of matters which have already well known and the repeated descriptions of substantially the same configurations may be omitted. This is because the following description is avoided from being redundant more than necessary, and facilitates the understanding by a person skilled in the art.

Note that the invertors provide the accompanying drawings and the following description to help a person skilled in the art fully understand the present disclosure, and are not intended to limit the subject matters described in the scope of claims thereby.

### (First Embodiment)

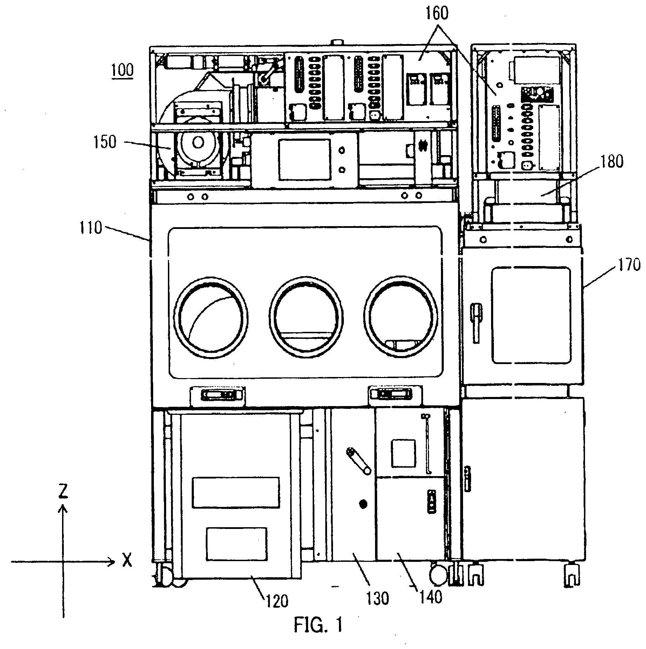

An isolator system 100 will be described hereinafter as an example of an isolator system in a first embodiment with reference to Figs. 1 to 12.

The isolator system 100 in the first embodiment is a device configured to perform, for example, work for cell culture, manipulation, observation, etc., in a sterilized environment. Sterilizing refers to an act of killing microorganisms, cells, etc., to bring a state closer to a sterile environment.

Note that, in the present embodiment, the Z-axis is an axis along a vertical direction in which the isolator system 100 is provided to stand, and it is assumed that a direction toward the upper side is +Z-direction and a direction toward the lower side (downward) is -Z-direction. The Y-axis is an axis along a direction perpendicular to the front surface and the back surface of the isolator system 100, and it is assumed that a direction extending from the front surface, where an opening for conducting work within a work space is provided, to the back surface opposite to the front surface is -Y-direction, and a direction extending from the back surface to the front surface is +Y-direction. The X-axis is an axis along a direction perpendicular to the side surfaces on the left and right sides when seen from the front, and it is assumed that a direction extending from the left side surface to the right side surface when seen from the front is +X-direction, and a direction extending from the right side surface to the left side surface is -X-direction.

### [1. Configuration]

### [1-1. Isolator System]

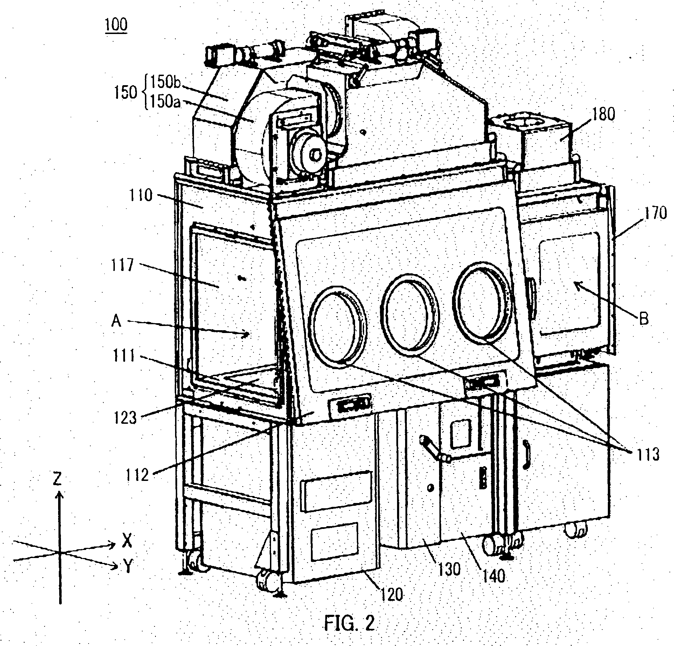

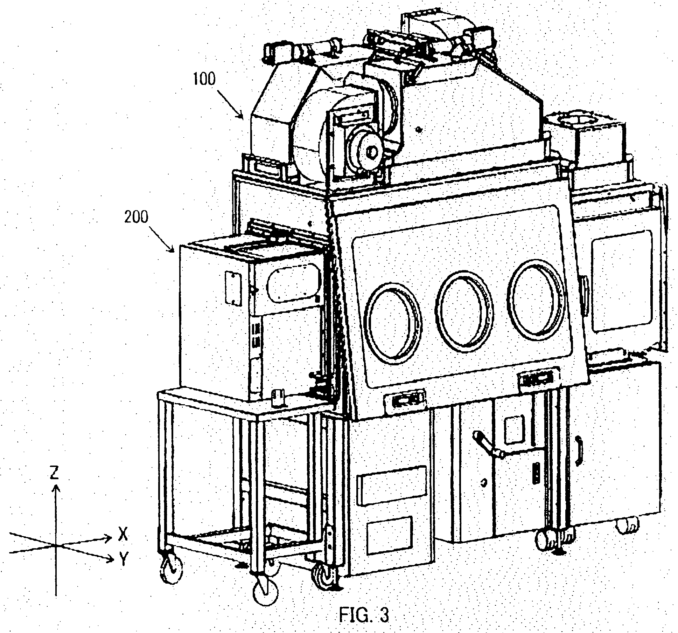

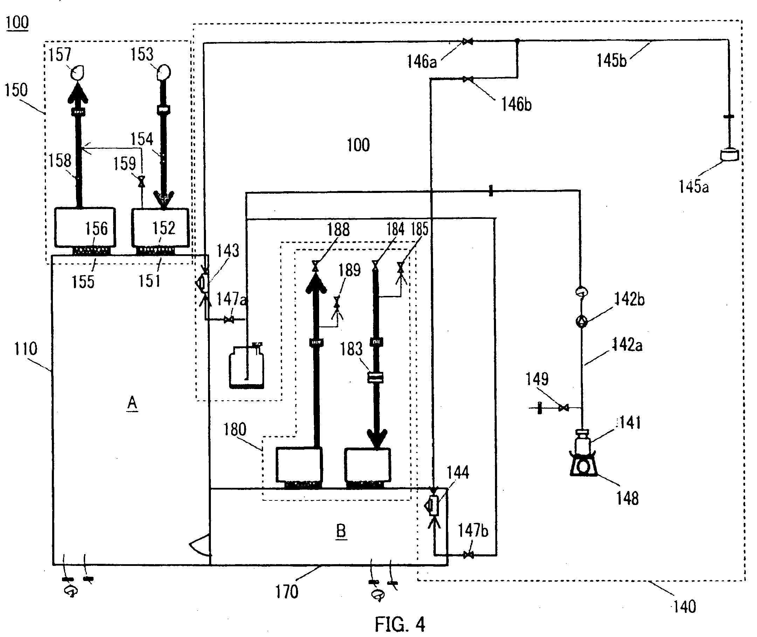

The overall configuration of the isolator system 100 will be described with reference to Figs. 1 to 4. Figs. 1 and 2 are a front view and a perspective view of the isolator system 100 according to the first embodiment, respectively. Fig. 3 is a perspective view of the isolator system 100 when an incubator 200 is mounted according to the first embodiment, and Fig. 4 is a schematic view illustrating the isolator system 100 according to the first embodiment.

As illustrated in Fig. 1, the isolator system 100 according to the first embodiment includes: an isolator 110; a centrifuge unit 120; an observation unit 130; a sterilizing unit 140; an air conditioning unit 150; a control unit 160; a pass box 170; and an air conditioning unit 180.

As illustrated in Fig. 2, the isolator 110 includes a substantially box-shaped-work space A that is isolated from the surroundings. The detailed configuration thereof will be described later. The centrifuge unit 120 is provided below the isolator 110, and can be connected from the work space A. The centrifuge unit 120 includes, in the interior thereof, a centrifuge 121 configured to centrifuge a sample to be worked within the work space A. The observation unit 130 is provided below the isolator 110, and can be connected from the work space A. The observation unit 130 includes, in the interior thereof, an observation device 131 configured to observe a sample to be worked on within the work space A. Further, the observation unit 130 includes: an elevating mechanism 133 capable of raising and lowering the observation device 131 provided therewithin; and a handle 132 with which the elevating mechanism 133 provided outside is operated. A worker operates the elevating mechanism 133 with the handle 132, thereby being able to lift the observation device 131 into the work space A when the observation device 131 is used, and accommodate the observation device 131 in the observation unit 130 when the observation device 131 is not used. The sterilizing unit 140 is provided below the isolator 110 in order to sterilize the interior of the isolator 110. The sterilizing unit 140 is configured to spray a sterilizing mist, obtained by converting a sterilizing liquid into a mist form, form through a nozzle 143, which is provided within the isolator 110, or a nozzle 144, which is provided within the pass box, and sterilize the interior thereof.

The air conditioning unit 150 is provided outside and above the isolator 110 and is configured to control the air conditioning therewithin. The air conditioning unit 150 includes an intake unit 150a and a discharge unit 150b. The air conditioning unit 150 includes an inlet 151 and an outlet 155 provided on an upper surface plate in the isolator 110. The air is supplied into the isolator 110 from the inlet 151, and is discharged from the outlet 155. In the isolator 110, in order to secure a sterile environment in the interior thereof, a particulate trap filter of a filter 152 is provided to the inlet 151, and the air is supplied through the particulate trap filter into the isolator 110. A filter 156 is also provided to the outlet 155, and the gas within the isolator 110 is discharged through the filter 156 from the interior of the isolator 110. In the isolator 110, a sterilizing substance such as hydrogen peroxide is sprayed thereinto, and thereby sterilizing processing of sterilizing the interior of the isolator 110 is performed. For example, a HEPA (High-Efficiency Particulate Air filter) may be used as the filter.

The control unit 160 is provided above the isolator 110 and the pass box 170, and is configured to control the operations of devices such as the sterilizing unit 140, the air conditioning unit 150, a plurality of diffusion fans 190, and the like, which will be described later.

The pass box 170 is provided on the side surface of the isolator 110 so that a worker puts a work item into the work space A from the outside. In the interior of the pass box 170, a transport space B for temporarily storing the work item is formed. The transport space B has hermeticity against the surrounding environment. Before the work item is put into the work space A from the outside, the work item is sterilized within the transport space B. In the side surface of the pass box 170, an opening for moving the work item is provided, and the isolator 110 and the pass box 170 are fixed so that the opening in the side surface of the pass box 170 is opposed to an opening provided in the side surface of the isolator 110. Thus, the work space A and the transport space B communicate with each other with hermeticity being maintained. A door that is openable and closable is mounted to the opening of the pass box 170. The door can separate the transport space B and the work space A with hermeticity being maintained. The air conditioning unit 180 is provided outside and above the pass box 170 and is configured to control the air conditioning within the transport space B.

As illustrated in Fig. 2, in the isolator system 100, the incubator 200 is mounted on the side surface opposite to the side surface of the isolator 110 to which the pass box 170 is provided. The incubator 200 includes a storage chamber (not illustrated) in the interior thereof. The storage chamber is a chamber in which a culture is stored, and is partitioned as a space that restrains bacterial invasion from the outside by, for example, a rectangular parallelepiped-shaped box body. The storage chamber is partitioned with, for example, stainless steel plates. The incubator 200 is demountably configured with respect to the isolator system 100. As a result, culture can be controlled in each incubator 200. For example, a dedicated incubator 200 is used for each donor, and thus it is possible to restrain occurrence of failures such as mix-ups of cultures.

### [1-2. Isolator]

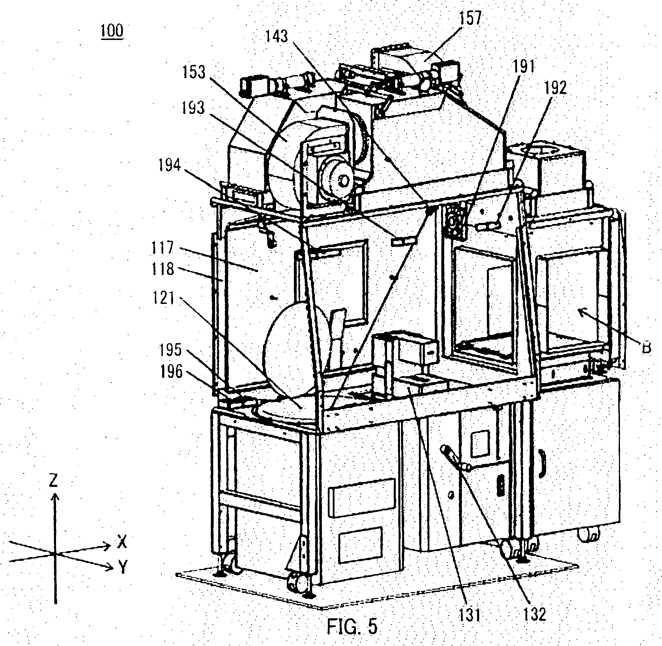

The configuration of the isolator 110 will be described with reference to Figs. 2, 4 and 5. Fig. 5 is a partial perspective view illustrating the internal configuration of the isolator system 100 according to the first embodiment.

As illustrated in Fig. 2, the isolator 110 is partitioned by a front surface plate 112 provided with a plurality of front surface openings 113, a bottom surface plate, an upper surface plate and side surface plates on the left and right sides, and includes, in the interior thereof, a work platform 111 used for conducting work; and a partition plate 117. The work space A in a box shape, which is a space for conducting work, is partitioned by the work platform 111, the partition plate 117, the upper surface plate, and the left and right side surface plates. The isolator 110 is partitioned to have hermeticity so that bacterial invasion from the outside is restrained. A duct 118 for discharging the gas in the work space A is formed between the work platform 111 and the bottom surface plate and between the back surface plate and the partition plate 117. In the isolator 110 of the present embodiment, the work platform 111, the partition plate 117, the bottom surface plate, the back surface plate, the upper surface plate, and the left and right side surface plates are configured with stainless steel plates, which are easily cleaned and sterilized.

The front surface plate 112 is configured with a glass plate, and the front surface thereof is provided with a plurality of openings 113 that are insertion portions for workers' hands. A glove (not illustrated) is mounted in each of the openings 113. The front surface plate 112 is openable and closable with respect to a hinge, serving as an axis, provided in an upper end portion. Thus, the front surface of the isolator 110 is openable and closable. In the left and right side surface plates, openings for mounting the pass box 170 and the incubator 200 are provided. In the isolator 110, the pass box 170 is mounted to the opening of the right side surface plate, and a door for a delivery opening of the pass box 170 constitutes a part of the right side surface plate. Further, the incubator 200 is mounted to the left opening of the left side surface plate, and a door of the incubator 200 constitutes a part of the left side surface plate. In working, the worker conducts work in the work space A through the glove. Hanging bars, on which items used for work are hung, are provided on the upper surface plate.

In the work platform 111, a connection opening portion for connecting to the centrifuge unit 120 is formed, and a cover 123 which can open and close the connection opening portion is provided. The cover 123 is mounted to the work platform 111 with a hinge, and can be opened upward and closed. A worker opens the cover 123 from the work space A, and thereby can connect to the centrifuge 121. Similarly, a connection opening portion for connecting to the observation unit 130 is formed in the work platform 111, and the platform includes a cover that can open and close the connection opening portion. The cover is mounted to the work platform 111 with a hinge, and can be opened upward and closed. A worker opens the cover from the work space A, and thereby can connect to the observation unit 130. When the worker uses the observation device 131, the worker opens the cover upward to connect to the observation unit 130, elevates and lowers the observation device 131 in the observation unit 130 with the elevating mechanism 133, and uses the observation device 131.

### [1-3. Sterilizing Unit]

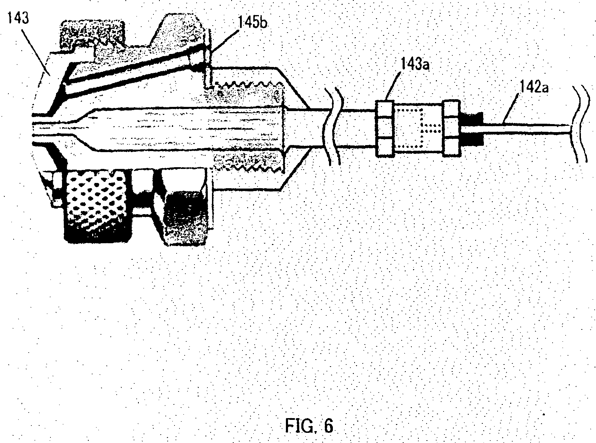

The configuration of the sterilizing unit 140 will be described with reference to Figs. 4 to 6. Fig. 6 is a schematic view illustrating the configuration of the nozzle 143 according to the first embodiment. In the present embodiment, as will be described later, the sterilizing unit 140 uses a spray method to convert the sterilizing liquid into mist and sprays it from the nozzle 143 or the nozzle 144.

As illustrated in Fig. 4, the sterilizing unit includes: a sterilizing liquid bottle 141; an electronic balance 148 configured to measure the weight of the sterilizing liquid bottle 141; and a water suction tube 142a and a peristaltic pump 142b for suctioning the sterilizing liquid from the sterilizing liquid bottle 141 and supplying it to the nozzle 143 or the nozzle 144. The sterilizing unit 140 also includes: the nozzle 143 provided within the isolator 110; and the nozzle 144 provided within the pass box 170. The sterilizing unit 140 also includes a compressor 145a and an intake tube 145b for supplying air to the nozzle 143 or the nozzle 144. The sterilizing unit 140 also includes: an intake valve 149 for introducing the air into the water suction tube 142a. The intake valve 149 is provided between the tip of the water suction tube 142a inserted into the sterilizing liquid bottle 141 and the peristaltic pump 142b. A switching valve 146a and a switching valve 146b are opened/closed, thereby controlling the flow of the air into the nozzle 143 or the nozzle 144. A switching valve 147a and a switching valve 147b are opened/closed, thereby controlling the flow of the sterilizing liquid into the nozzle 143 or the nozzle 144.

For example, a hydrogen peroxide solution is used as the sterilizing liquid. The sterilizing liquid is stored within the sterilizing liquid bottle 141, and the sterilizing liquid bottle 141 is placed on a measurement means such as the electronic balance 148. The electronic balance 148 is configured to measure the weight of the sterilizing liquid bottle 141 and the sterilizing liquid, and its signal is inputted to the control unit 160. The sterilizing liquid stored in the sterilizing liquid bottle 141 is supplied by a liquid delivery device, such as the peristaltic pump 142b, through the water suction tube 142a to the nozzle 143 or the nozzle 144. The hydrogen peroxide solution in the sterilizing liquid bottle 141 is supplied in a gradual manner by the peristaltic pump 142b to the nozzle 143 or the nozzle 144. The peristaltic pump 142b is provided somewhere in the water suction tube 142a, and a filter configured to remove foreign substances is provided on the downstream side with respect to the peristaltic pump 142b. A pressure sensor configured to detecting the pressure within the water suction tube 142a is provided between the filter and the peristaltic pump 142b. With the pressure sensor, it is possible to monitor the clogging of the filter or detect abnormalities such as bending of the water suction tube 142a.

Since signals from the electronic balance 148 and the pressure sensor are input to the control unit 160, the control unit 160 can control the driving of the peristaltic pump 142b and the opening and closing of the intake valve 149.

Further, one end of the intake tube 145b is attached to the compressor 145a, and the other end thereof is attached to the nozzle 143 or the nozzle 144. Thereby, the compressor 145a can supply air through the intake tube 145b to the nozzle 143 or the nozzle 144.

As illustrated in Fig. 5, the nozzle 143 is mounted to the corner on the right side (+X-side) in an upper portion (+Z-side) on the back surface side (-Y-side) in the isolator 110. The nozzle 143 can spray the sterilizing mist, obtained by converting a sterilizing liquid into mist, toward the corner on the left side (-X-side) in a lower portion (-Z-side) on the front surface side (+Y-side).

As illustrated in Fig. 6, the nozzle 143 includes a path for the sterilizing liquid and a path for the air. The path for the sterilizing liquid in the nozzle 143 is connected through a joint 143a to the water suction tube 142a. The path for the sterilizing liquid is shifted, by the joint 143a, from the water suction tube 142a having a small inside diameter to the nozzle 143 having a large inside diameter. In other words, the inside diameter of the path is increased within the joint 143a. Further, in the nozzle 143, the intake tube 145b is connected to the path for the air which is different from the path for the sterilizing liquid, and the air is ejected from the end of the nozzle 143. Since the ejection port of the air is formed in such a manner as to surround the ejection port of the sterilizing liquid, the air in a doughnut-shape is ejected from the nozzle 143. Hence, a negative pressure is created around the center of the air, and thus the sterilizing liquid passing through the water suction tube 142a is drawn into and diffused at the end of the nozzle 143, thereby being converted into mist.

Note that since the configuration of the nozzle 144 is substantially the same as that of the nozzle 143, the description thereof will be omitted.

In such a configuration, the hydrogen peroxide solution supplied to the nozzle 143 or the nozzle 144 is converted into mist by air supplied to the nozzle 143 or the nozzle 144 and is sprayed into the isolator 110 or the pass box 170.

Here, in the present embodiment, the flow rate of the sterilizing liquid supplied by the peristaltic pump 142b is set lower than the flow rate of the sterilizing liquid drawn into the nozzle 143 by the creation of a negative pressure within the nozzle 143 through the delivery of the air with the compressor 145a. Note that, in the present embodiment, the flow rate of the sterilizing liquid supplied by the peristaltic pump 142b is about 2 ml/min, and the flow rate of the sterilizing liquid drawn into the nozzle 143 by the creation of a negative pressure within the nozzle 143 is about 8 ml/min.

Thus, since the air supply is larger than the sterilizing liquid supply, the sterilizing mist can be intermittently sprayed from the nozzle 143 or the nozzle 144.

### [1-4. Diffusion Fan]

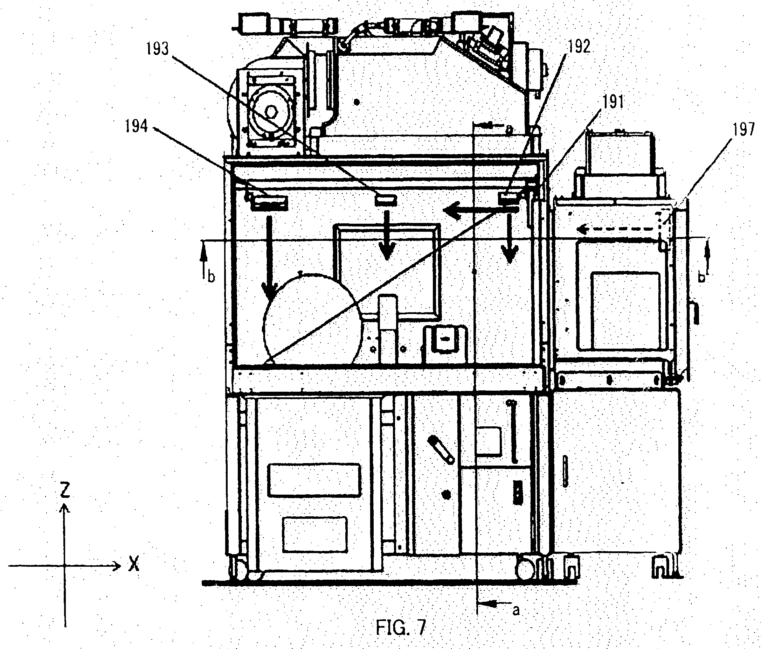

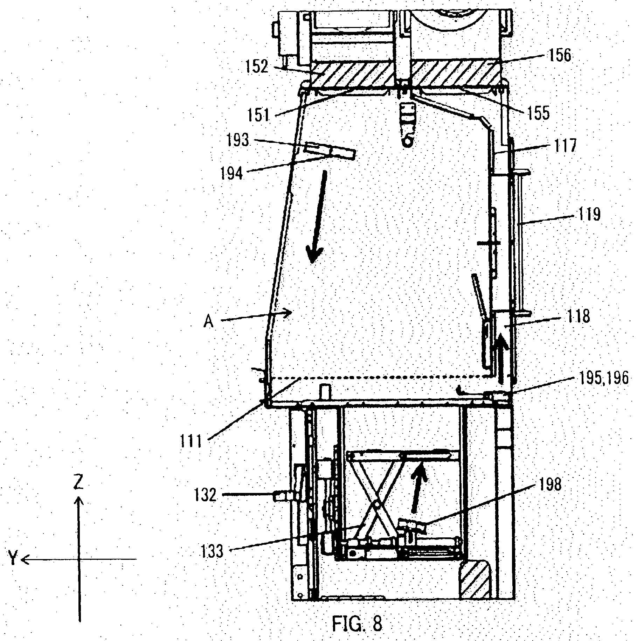

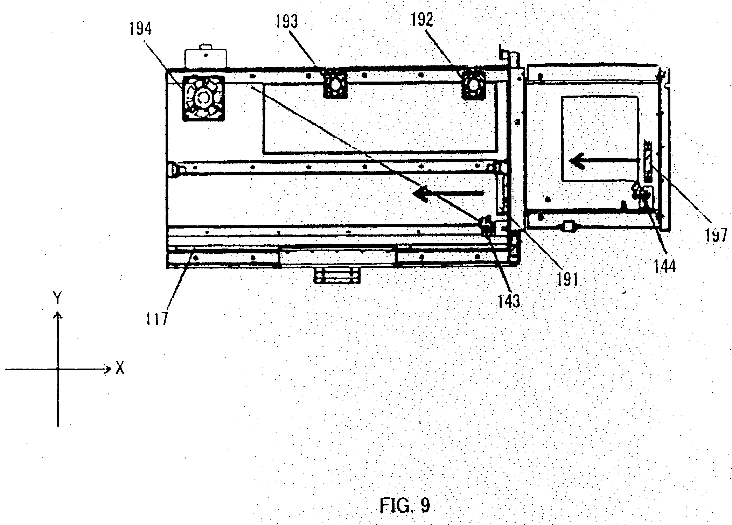

The configuration of the plurality of diffusion fans 190 will be described with reference to Fig. 5 and Figs. 7 to 9. Fig. 7 is a partial front view illustrating the internal configuration of the isolator system 100 according to the first embodiment, Fig. 8 is a cross-sectional view taken along line a-a in Fig. 7 and Fig. 9 is a cross-sectional view taken along line b-b in Fig. 7.

The isolator system 100 includes the plurality of diffusion fans 190. Specifically, the diffusion fans 191 to 196 are provided within the isolator 110 (Fig. 5), the diffusion fan 197 is provided within the pass box 170 (Fig. 7), and the diffusion fan 198 is provided within the observation unit 130 (Fig. 8).

Firstly, the diffusion fans 191 to 196 within the isolator 110 will be described. As illustrated in Fig. 5, the diffusion fan 191 is provided in an upper portion on the back surface side on the right side surface side of the isolator 110, and generates air current from the right side to the left side (-x-direction).

### [2. Operation]

### [2-1. Control Unit]

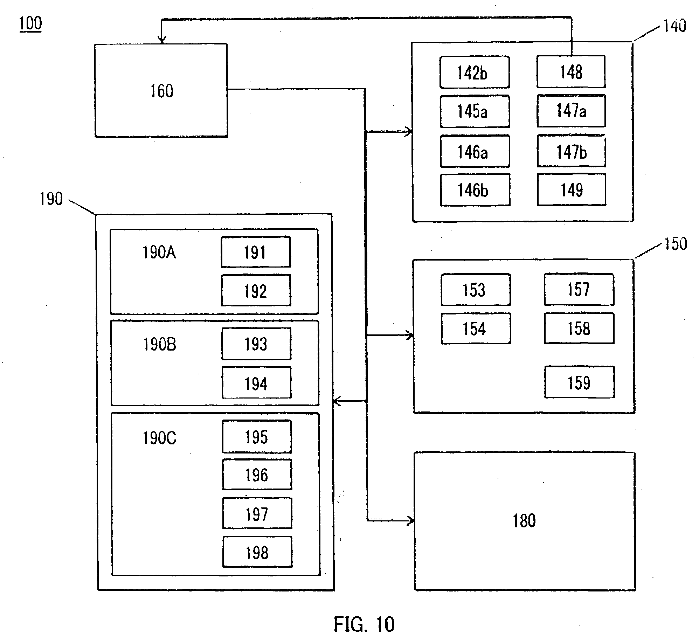

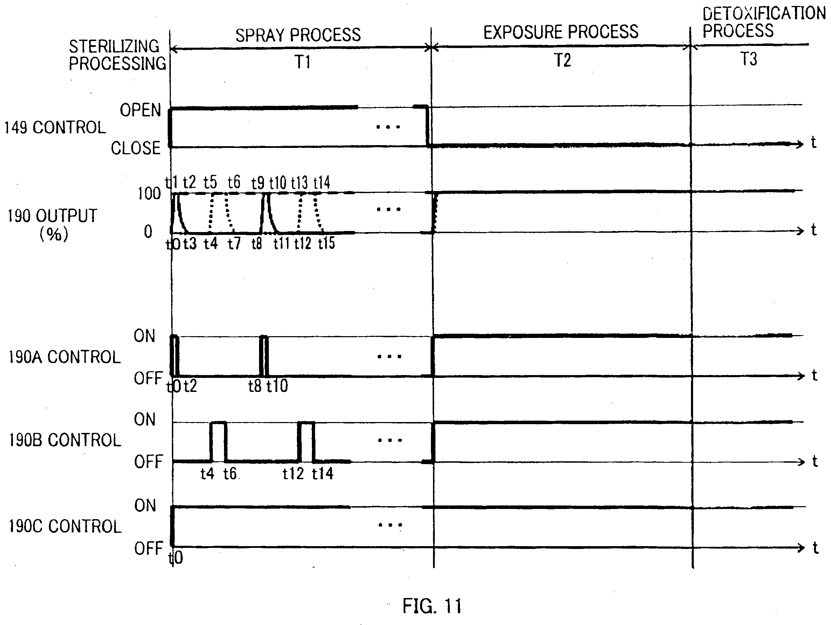

The control of the control unit 160 will be described with reference to Figs. 10 and 11. Fig. 10 is a block diagram illustrating an example of the control of the control unit 160 according to the first embodiment. Fig. 11 is a timing chart illustrating an example of the control of the control unit 160 according to the first embodiment.

As illustrated in Fig. 10, the control unit 160 outputs signals to the sterilizing unit 140, the air conditioning unit 150, the air conditioning unit 180, and the plurality of diffusion fans 190. A signal is outputted to each of the components of the sterilizing unit 140 in response to an input from the electronic balance 148, etc. The control unit 160 performs a spray process, an exposure process and a detoxification process, as the sterilizing processing for sterilizing the interior of the isolator 110. The spray process is a process of spraying the sterilizing mist in the interior of the isolator 110. The exposure process is a process of diffusing the sprayed sterilizing mist over the entire interior of the isolator 110. The detoxification process is a process of supplying the air into the interior of the isolator 110 while discharging the gas in the isolator 110 and thereby exchanging the gas in the isolator 110.

### [2-2. Spray Process]

As illustrated in Fig. 11, in the spray process, the control unit 160 causes the sterilizing unit 140 to operate so as to spray the sterilizing mist in the interior of the isolator 110. Further, at the same time, the control unit 160 controls the operations of the air conditioning unit 150 and the plurality of diffusion fans 190. In the present embodiment, a time period T1 required for the spray process is 15 minutes.

### <Sterilizing Unit>

Firstly, before the spray process is performed, the control unit 160 causes the electronic balance 148 to measure the total weight of the sterilizing liquid bottle 141 and the sterilizing liquid, in a state where the peristaltic pump 142b is stopped and the intake valve 149 is opened. Then, as illustrated in Fig. 11, in the spray process, the control unit 160 starts the operation of the sterilizing unit 140. The control unit 160 causes the intake valve 149 to be closed, and causes the peristaltic pump 142b and the compressor 145a to start up. Thus, when the sterilizing liquid in the sterilizing liquid bottle 141 is suctioned into the water suction tube 142a, the weight measured is reduced by way of the intake valve 149 accordingly. The sterilizing liquid in the sterilizing liquid bottle 141 is supplied, by every predetermined amount, via the water suction tube 142a and the filter to the nozzle 143 through the operation of the peristaltic pump 142b. In the present embodiment, it is about 2 ml/min. Here, while the sterilizing unit 140 is being operated, the sterilizing unit 140 intermittently sprays the sterilizing,mist.

When the weight measured by the electronic balance 148 is reduced to the predetermined amount, the control unit 160 causes the intake valve 149 to be opened to introduce the air as a gas to the water suction tube 142a. Thus, the sterilizing liquid on the downstream side with respect to the intake valve 149 is supplied to the nozzle 143 by the peristaltic pump 142b, whereas the sterilizing liquid on the upstream side with respect to the intake valve 149 is returned into the sterilizing liquid bottle 141.

### <Diffusion Fan>

In the spray process, the control unit 160 drives the plurality of diffusion fans 190 to diffuse and evaporate the sterilizing mist. As illustrated in Fig. 11, the control unit 160 causes a first diffusion fan group 190A including the diffusion fans 191 and 192 and a second diffusion fan group 190B including the diffusion fans 193 and 194 to operate in an alternate manner. The control unit 160 causes at least two diffusion fans of the diffusion fan 191 and diffusion fan 194 whose airflow directions are different from each other to operate intermittently. The first diffusion fan group 190A first starts operating concurrently with the start of the spray process (t0), and the operation is maintained for a predetermined time period, e.g., 4 seconds (t2-t1), after the output thereof reaches 100% (t1). Thereafter, the first diffusion fan group 190A is stopped (t2), the output is lowered over a predetermined time period, e.g., about 10 seconds, and the output reaches 0% (t3). The second diffusion fan group 190B starts operating (t4), when a predetermined time period, for example, 10 seconds has elapsed after the output of the first diffusion fan group 190A reaches 0%. In the second diffusion fan group 190B, after the output thereof reaches 100% (t5), the operation thereof is maintained for a predetermined time period, e.g., 10 seconds (t6-t5). Thereafter, the second diffusion fan group 190B is stopped (t6), the output is lowered over a predetermined time, e.g., about 10 seconds, and the output thereof reaches 0% (t7). Thereafter, the first diffusion fan group 190A starts operating (t8), when a predetermined time period, i.e., 15 seconds, has elapsed after the output of the second diffusion fan group 190B reaches 0%. In other words, after being stop, the first diffusion fan group 190A has been stopped for a predetermined time period, i.e., 45 seconds, until the subsequent start. Similarly, the second diffusion fan group 190B has been stopped for a predetermined time period, i.e., 40 seconds, after being stopped until the subsequent start. As such, in the first diffusion fan group 190A and the second diffusion fan group 190B, each operating time thereof is shorter than each stop time thereof.

In the first diffusion fan group 190A and the second diffusion fan group 190B, such control is performed by the control unit 160 in predetermined cycles, i.e., 15 cycles, in the spray process.

The control unit 160 is configured to cause a third diffusion fan group 190C including the diffusion fans 195 to 198 to start operating concurrently with the start of the spray process, and maintains its continuous operation.

### <Air Conditioning Unit>

In the spray process, the control unit 160 is configured to cause a back-flow valve 159 to be opened/closed based on an input from the pressure sensor, which is configured to detect the pressure within the isolator 110, and adjust the pressure within the isolator 110. When the sterilizing mist is sprayed in the isolator 110 by the sterilizing unit 140, the interior of the isolator 110 is brought into a pressurized state, and thus when a predetermined pressure is reached, the control unit 160 causes the back-flow valve 159 to be released. Here, since a back-flow path in which the back-flow valve 159 is provided is connected to the inlet 151, the sterilizing mist or the sterilizing gas within the isolator 110 is discharged through the filter 152, thereby being able to perform the sterilizing processing for the filter 152. Further, in the spray process, the pressure within the isolator 110 may be released from an upper portion in the isolator 110.

### [2-3. Exposure Process]

Next, in the exposure process, the control unit 160 causes the sterilizing mist sprayed in the spray process and the sterilizing gas obtained by evaporating the sterilizing mist to be diffused, and sterilizes the interior of the isolator 110. As illustrated in Fig. 11, in the exposure process, the control unit 160 stops the operation of the sterilizing unit 140, causes the plurality of diffusion fans 190 to be driven and causes the sterilizing mist sprayed into the isolator 110 or the evaporated sterilizing gas to be diffused. At the same time, the control unit 160 controls the operation of the plurality of diffusion fans 190. In the present embodiment, a time period T2 required for the exposure process is 15 minutes.

### <Sterilizing Unit>

As illustrated in Fig. 11, the control unit 160 causes the peristaltic pump 142b and the compressor 145a to continue operating in the exposure process, even after the completion of the supply of the sterilizing liquid within the water suction tube 142a to the isolator 110. In other words, after the end of the spray process, the operation of the sterilizing unit is continued.

### <Diffusion Fan>

In the exposure process, the control unit 160 causes the plurality of diffusion fans 190 to be driven to diffuse the sterilizing mist and the sterilizing gas within the isolator 110, thereby sterilizing the interior of the isolator 110. As illustrated in Fig. 11, concurrently with the start of the exposure process, all of the plurality of diffusion fans 190 starts operating. In other words, the control unit 160 causes the first diffusion fan group 190A, the second diffusion fan group 190B and the third diffusion fan group 190C to start operating. Then, during the exposure process, the control unit 160 causes the plurality of diffusion fans 190 to continue operating.

### <Air Conditioning Unit>

In the exposure process, the control unit 160 causes the back-flow valve 159 to be closed concurrently with the end of the spray process, and thereafter causes the air conditioning unit 150 not to operate. For example, in the exposure process, the control unit 160 may continue the same operation as in the spray process.

### [2-4. Detoxification Process]

Finally, in the detoxification process, the control unit 160 causes the air conditioning unit 150 to be driven to supply the air into the isolator 110 while discharging the gas within the isolator 110, thereby exchanging the gas within the isolator 110. In the present embodiment, a time period T3 required for the detoxification process is 30 minutes.

### <Sterilizing Unit>

As illustrated in Fig. 11, in the detoxification process, the control unit 160 causes the compressor 145a to continue operating and reverses the operation of the peristaltic pump 142b. Thus, the sterilizing liquid left within the water suction tube 142a is collected.

### <Diffusion fan>

In the detoxification process, the control unit 160 causes the plurality of diffusion fans 190 to be driven to diffuse the sterilizing gas within the isolator 110, and assists the exchange of the sterilizing gas within the isolator 110. As illustrated in Fig. 11, the control unit 160 causes all of the plurality of diffusion fans 190 operating in the exposure process to continue operating. In other words, the control unit 160 causes the first diffusion fan group 190A, the second diffusion fan group 190B and the third diffusion fan group 190C to continue operating. Then, during the detoxification process, the control unit 160 causes the plurality of diffusion fans 190 to continue operating.

### <Air Conditioning Unit>

In the detoxification process, the control unit 160 causes the air conditioning unit 150 to be driven to supply the air taken in through an intake blower 153 into the isolator 110, and thereby the sterilizing gas within the isolator 110 is pushed out, and the gas within the isolator 110 is exchanged. Firstly, the control unit 160 causes the back-flow valve 159 to be closed concurrently with the end of the exposure process, and causes an intake valve 154 and a discharge valve 158 to be released. Then, the control unit 160 causes the intake blower 153 and a discharge blower 157 to operate. Then, during the detoxification process, the control unit 160 causes the intake blower 153 and the discharge blower 157 to continue operating, with the back-flow valve 159 being closed,.

As described above, the sterilizing processing in the present embodiment is performed. The total period of time required for the sterilizing processing in the present embodiment is about 1 hour, and time reduction can be achieved as compared with a conventional device.

### [3. Effects, etc.]

As described above, in the present embodiment, the isolator system 100 (an example of the isolator) includes: the box-shaped isolator 110 (an example of a main body case) that includes the work space A isolated from the surroundings; the sterilizing unit 140 (an example of a spray device) configured to spray the sterilizing mist from the nozzle 143 mounted within the isolator 110, the sterilizing mist being obtained by converting a sterilizing liquid into mist; and the control unit 160 (an example of a control device) configured to control the operation of the sterilizing unit 140. The sterilizing unit 140 configured to intermittently spray the sterilizing mist when spraying.

Thus, since a period of time required for warming up the heater is not necessary as compared with a conventional one, a sterilizing processing time can be reduced. Further, since being intermittently spayed, the sterilizing mist is not continuously sprayed to the inner wall surface of the isolator 110, and thus the inner wall surface is intermittently brought into contact with the gas. As a result, it is possible to restrain the particulates of the sterilizing liquid, which is produced by adhesion of the sterilizing mist to the inner wall surface of the isolator 110, from growing into large droplets. Hence, even if the sterilizing liquid still in a mist state is sprayed without being evaporated in the spray process, it is possible to reduce a period of time required for evaporating the droplets of the sterilizing mist. Thus, at least a period of time required for the detoxification process can be reduced, with the result that it is possible to reduce a period of time required for the sterilizing processing can be reduced.

In the present embodiment, the sterilizing unit 140 is configured to spray the sterilizing mist, obtained by converting the sterilizing liquid into mist, substantially diagonally, from the nozzle 143 mounted on the right side (+X-side) in an upper portion (+Z-side) on the back surface side (-Y-side) within the isolator 110 toward the left side (-X-side) in a lower portion (- Z-side) on the front surface side (+Y-side).

Thus, since a part, onto which the sterilizing mist is sprayed from the nozzle 143, in the inner wall surface of the isolator 110 can be kept away from the nozzle 143, it is possible to restrain droplets from growing on the inner wall surface of the isolator 110. Although, in the present embodiment, the sterilizing mist is sprayed substantially diagonally from the nozzle 143, which is mounted on the right side in the upper portion on the back surface side within the isolator 110, toward the left side in the lower portion on the front surface side, the nozzle 143 may be arranged in another position as long as the sterilizing mist can be sprayed substantially diagonally.

In the present embodiment, the sterilizing unit 140 includes: the water suction tube 142a (an example of the water suction tube) through which the sterilizing liquid is suctioned; the peristaltic pump 142b (an example of the liquid delivery device) configured to adjust the flow rate of the sterilizing liquid to be suctioned; the nozzle 143 (an example of the nozzle) configured to convert the suctioned sterilizing liquid into mist; and the compressor 145a (an example of the compressor) configured to deliver the air into the nozzle 143 and cause the sterilizing liquid to be sprayed from the nozzle 143. The flow rate in the peristaltic pump 142b is set lower than the flow rate of the sterilizing liquid drawn into the nozzle 143 by the creation of a negative pressure within the nozzle 143 through the delivery of the air with the compressor 145a, so that the sterilizing unit 140 intermittently sprays the sterilizing mist.

Thus, the sterilizing unit 140 can intermittently spray the sterilizing mist. Further, since dripping from the nozzle 143 can be restrained, it is possible to restrain the sterilizing liquid from forming large droplets.

Further, in the present embodiment, the control unit 160 is configured to cause the spray process, in which the sterilizing unit 140 sprays the sterilizing mist, to be intermittently performed, and the sterilizing unit 140 is configured to intermittently spray the sterilizing mist in the spray process.

Thus, since not only the sterilizing mist is intermittently sprayed but also the spray process itself is intermittently performed as well, the sterilizing mist is not continuously sprayed onto the inner wall surface of the isolator 110, thereby being able to restrain the particulates of the sterilizing liquid, adhering to the inner wall surface of the isolator 110, from growing into large droplets. Thus, a period of time required for the sterilizing processing can be reduced.

Further, in the present embodiment, further included are: the intake unit 150a (an example of an intake device) configured to take gas into the isolator 110 through the inlet 151 (an example of the inlet) provided in an upper portion of the isolator 110; and the discharge unit 150b (an example of a discharge device) configured to discharge gas from the interior of the isolator 110 through the outlet 155 (an example of the outlet) provided in the upper portion of the isolator 110. When the sterilizing unit 140 is spraying, the pressure within the isolator 110 can be released from at least one of the inlet 151 and the outlet 155.

Thus, since the sterilizing mist heavier than gas can be raised upward, the sterilizing mist and the sterilizing gas within the isolator 110 are preferably dispersed. Hence, a period of time required for the spray process can be reduced, with the result that a period of time required for the sterilizing processing can be reduced.

Further, in the present embodiment, the diffusion fan 194 (an example of the diffusion fan), which is mounted within the isolator 110 to diffuse the sterilizing mist, is further included. The control unit 160 is configured to cause at least the diffusion fan 194 to intermittently operate while the sterilizing unit 140 is spraying, and cause at least the diffusion fan 194 to be continuously operating while the sterilizing unit 140 is stopped.

Hence, while the sterilizing mist is being sprayed, that is, while the sterilizing unit is spraying, at least the sterilizing mist is not continuously taken in by the diffusion fan 194 , and thus it is possible to restrain the small droplets of the sterilizing liquid, produced by adhesion of the sterilizing mist, from growing into the droplets of the sterilizing liquid. Further, since spraying to the inner wall surface within the isolator 110 is not continued, it is also possible to restrain the small droplets, adhering to the inner wall surface of the isolator 110, from growing. Hence, at least a period of time required for evaporating the droplets of the sterilizing mist can be reduced in the detoxification process. Thus, at least a period of time for the detoxification process can be reduced, with the result that a period of time required for the sterilizing processing can be reduced. Note that, in the present embodiment, not only the diffusion fan 194 but also the diffusion fans 191 to 193 are intermittently operated due to the same reasons. Here, although the diffusion fan 191 is described as an example, it is not limited to the diffusion fan 191, and for example, if only any one of the diffusion fans 192, 193 and 194 is provided, the effects described above are achieved. However, the diffusion fan 194, which can blow out the air toward the inner wall surface of the isolator 110 sprayed with the sterilizing mist, is effective since it can restrain the droplets on the inner wall surface of the isolator 110 from growing.

Further, in the present embodiment, the diffusion fan 191 and the diffusion fan 194 (an example of at least two diffusion fans) whose airflow directions are different from each other are included. The control unit 160 is configured to cause the diffusion fan 191 and the diffusion fan 194 to alternately operate, while the sterilizing unit 140 is spraying. In the present embodiment, although the first diffusion fan group 190A including the diffusion fan 191 and the second diffusion fan group 190B including the diffusion fan 194 are alternately operated, a plurality of diffusion fans and another plurality thereof may be alternately operated or three or more diffusion fans may be sequentially operated. In other words, it is only necessary that at least two diffusion fans are alternately operated.

Thus, it is possible to enhance the diffusivity of the sterilizing mist and the sterilizing gas within the isolator 110 and further restrain the droplets from growing. Hence, a period of time required for each process can be reduced, with the result that a period of time required for the sterilizing processing can be reduced.

Further, in the present embodiment, the isolator system 100 includes: the air conditioning unit 150 (an example of an air conditioning device) ; the diffusion fan 191 (an example of a first diffusion fan) that is mounted within the isolator 110 to diffuse the sterilizing mist; and the control unit 160 configured to control the operations of the sterilizing unit 140 and the diffusion fan 191. The control unit 160 is configured to cause the diffusion fan 191 to intermittently operate while the sterilizing unit 140 is spraying.

Thus, since a period of time required for warming up the heater is not necessary as compared with a conventional one, a period of time required for the sterilizing processing can be reduced. Further, since the sterilizing mist within the isolator 110 can be diffused by the diffusion fan 191, it is possible to restrain the small droplets of the sterilizing liquid, produced by adhesion of the sterilizing mist to the inner wall surface of the isolator 110, from growing into large droplets. Further, since the sterilizing mist is not continuously suctioned by the diffusion fan 191, it is possible to restrain the small droplets of the sterilizing liquid, produced by adhesion of the sterilizing mist to the diffusion fan 191, from growing into large droplets. Hence, a period of time required for evaporating the droplets of the sterilizing mist can be reduced at least in the detoxification process. Thus, at least a period of time required for the detoxification process can be reduced, with the result that a period of time required for the sterilizing processing can be reduced. Note that, in the present embodiment, not only the diffusion fan 191 but also the diffusion fans 192, 193 and 194 are intermittently operated due to the same reasons. Here, although the diffusion fan 191 is described as an example, it is not limited to the diffusion fan 191, and for example, if only any one of the diffusion fans 192, 193 and 194 is provided, the effects described above are achieved. However, the diffusion fan 194, which is capable of blowing out the air toward the inner wall surface of the isolator 110 sprayed with the sterilizing mist, is effective since it can restrain the droplets on the inner wall surface of the isolator 110 from growing.

Further, in the present embodiment, the diffusion fan 194 (an example of a second diffusion fan) whose airflow direction is different from that of the diffusion fan 191 is further included within the isolator 110, and the control unit 160 is configured to cause each of the diffusion fans 191 and 194 to intermittently operate while the sterilizing unit 140 is spraying.

Thus, since the sterilizing mist within the isolator 110 can be diffused by the diffusion fans 191 and 194 whose airflow directions are different to each other, it is possible to enhance the diffusivity of the sterilizing mist and the sterilizing gas within the isolator 110 and also restrain the small droplets of the sterilizing liquid, produced by adhesion of the sterilizing mist to the inner wall surface of the isolator 110, from growing into large droplets. Further, since the sterilizing mist is not continuously suctioned by the diffusion fans 191 and 194, it is possible to restrain the small droplets of the sterilizing liquid, produced by adhesion of the sterilizing mist to the diffusion fans 191 and 194, from growing into large droplets. Hence, a period of time required for evaporating the droplets of the sterilizing mist can be reduced at least in the detoxification process. Thus, at least a period of time required for the detoxification process can be reduced, with the result that a period of time required for the sterilizing processing can be reduced.

Further, in the present embodiment, the control unit 160 is configured to cause the diffusion fan 191 and the diffusion fan 194 to alternately operate while the sterilizing unit 140 is spraying. In the present embodiment, although the first diffusion fan group 190A including the diffusion fan 191 and the second diffusion fan group 190B including the diffusion fan 194 are alternately operated, a plurality of diffusion fans and another plurality thereof may be alternately operated or three or more diffusion fans may be sequentially operated. In other words, it is only necessary that at least two diffusion fans are alternately operated.

Thus, it is possible to enhance the diffusivity of the sterilizing mist and the sterilizing gas within the isolator 110 and further restrain the droplets from growing. Hence, a period of time required for evaporating the droplets of the sterilizing mist can be reduced at least in the detoxification process. Thus, at least a period of time required for the detoxification process can be reduced, with the result that a period of time required for the sterilizing processing can be reduced.

Further, in the present embodiment, the control unit 160 is configured to, while the sterilizing unit 140 is spraying, cause the operation of the diffusion fan 194 to be stopped while the diffusion fan 191 is operating, and cause the operation of the diffusion fan 191 to be stopped while the diffusion fan 194 is operating.

Thus, even when the operating time of the diffusion fans 191 and 194 is set shorter, it is possible to operate one while the other is stopped, and therefore it is possible to enhance the diffusivity of the sterilizing mist and the sterilizing gas within the isolator 110 and further restrain the droplets from growing. Hence, a period of time required for each process can be reduced, with the result that a period of time required for the sterilizing processing can be reduced.

Further, in the present embodiment, the control unit 160 is configured to cause each operating time of the diffusion fans 191 and 194 to be shorter than a stop time, while the sterilizing unit 140 is spraying.

Thus, since the operating times of the diffusion fans 191 and 194 can be set shorter, it is possible to further restrain the droplets from growing. Hence, a period of time required for each process can be reduced, with the result that a period of time required for the sterilizing processing can be reduced.

Further, in the present embodiment, the diffusion fan 191 is configured to blow out the air from the right side (an example of one of the left and right sides) to the left side (an example of the other of the left and right sides) within the isolator 110, and the diffusion fan 194 is configured to blow out the air from the upper side to the lower side within the isolator 110.

Thus, it is possible to enhance the diffusivity of the sterilizing mist and the sterilizing gas within the isolator 110 and further restrain the droplets from growing. Hence, a period of time required for each process, with the result that a period of time required for the sterilizing processing can be reduced.

Further, in the present embodiment, the sterilizing unit 140 is configured to spray the sterilizing mist substantially diagonally from the nozzle 143 mounted on the right side (+X-side) in an upper portion on the back surface side (-Y-side) within the isolator 110 toward the left side (-X-side) in a lower portion on the front surface side (+Y-side), and the diffusion fan 194 is configured to blow out the air from an upper portion on the left side to a lower portion on the front surface side within the isolator 110.

Thus, since a part, onto which the sterilizing mist is sprayed from the nozzle 143, of the inner wall surface of the isolator 110 can be kept away from the nozzle 143, it is possible to restrain the droplets from growing on the inner wall surface of the isolator 110. Further, it is possible to blow the gas, which is blown out from the diffusion fan 194, toward the part onto which the sterilizing mist is sprayed from the nozzle 143. Hence, it is possible to promote the diffusivity of the gas and the evaporation of the droplets at the part, on which the droplets are easily grown and the sterilizing mist is sprayed, and to restrain the droplets from growing. Thus, it is possible to reduce a period of time required for each process, with the result that a period of time required for the sterilizing processing.

Further, in the present embodiment, the sterilizing unit 140 includes: the water suction tube 142a through which the sterilizing liquid is suctioned; the peristaltic pump 142b configured to adjust the flow rate of the sterilizing liquid to be suctioned; the nozzle 143 configured to spray the suctioned sterilizing liquid; and the compressor 145a configured to deliver the air into the nozzle 143, and spray the sterilizing liquid in a mist form from the nozzle 143. The flow rate in the peristaltic pump 142b is set lower than the flow rate of the sterilizing liquid drawn into the nozzle 143 by the creation of a negative pressure within the nozzle 143 through the delivery of the air with the compressor 145a, and thus the sterilizing unit 140 intermittently sprays the sterilizing mist.

Thus, the sterilizing unit 140 can intermittently spray the sterilizing mist. Further, since dripping from the nozzle 143 can be restrained, it is possible to restrain the sterilizing liquid from forming large droplets.

Further, in the present embodiment, included are the intake unit 150a configured to take gas into the isolator through the inlet 151 provided in an upper portion of the isolator 110; and the discharge unit 150b configured to discharge gas from the interior of the isolator 110 through the outlet 155 provided in an upper portion of the isolator 110. It is possible to release the pressure within the isolator 110 from at least one of the inlet 151 and the outlet 155 while the sterilizing unit 140 is spraying.

Thus, since the sterilizing mist heavier than gas can be raised upward, the sterilizing mist and the sterilizing gas within the isolator 110 are favorably dispersed. Hence, a period of time required for the spray process can be reduced, with the result that a period of time required for the sterilizing processing can be reduced.

### (Other Embodiments)

As described above, the first embodiment has been described as exemplification of technologies disclosed in the present application. However, the technologies in the present disclosure is not limited thereto, and can be applied to embodiments to which modification, replacement, addition, omission and/or the like is made as necessary. Further, it is also possible to provide new embodiments by combining the constituent elements described in the above first embodiment.

Hence, another embodiment will be exemplified hereinafter.

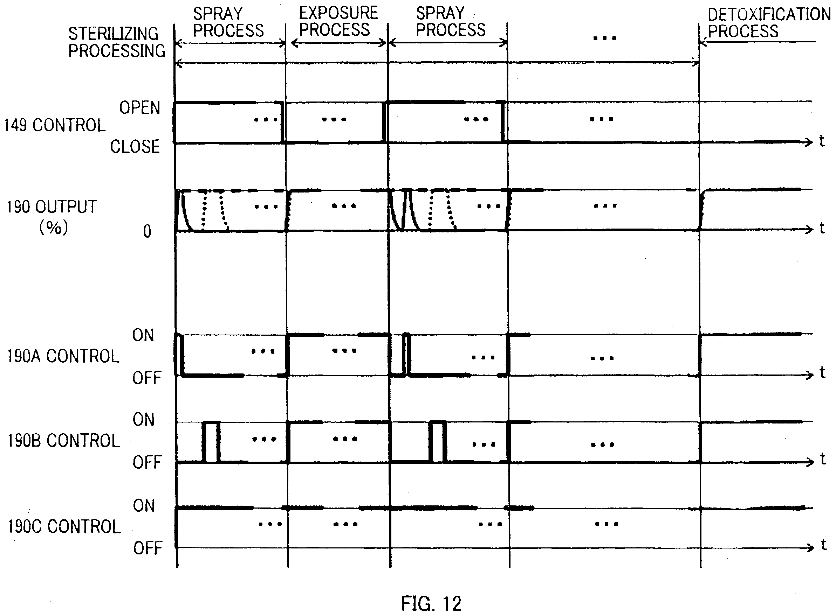

Fig. 12 is a timing chart illustrating an example of control of the control unit 160 according to another embodiment.

In the first embodiment, control illustrated in Fig. 11 has been described as an example of the control of the control unit 160. Specifically, in the first embodiment, the control unit 160 is configured to cause the sterilizing unit 140 to operate so as to spray in the spray process, and cause the sterilizing unit 140 to stop spraying in the exposure process. However, the control of the control unit 160 is not limited to the control illustrated in Fig. 11. As illustrated in Fig. 12, the control unit 160 may control the sterilizing unit 140 so as to intermittently perform the spray process. In such a case, the sterilizing unit 140 intermittently sprays the sterilizing mist in the spray process.

Thus, it is possible to restrain the droplets of the sterilizing mist, adhering to the inner wall surface of the isolator 110 and the plurality of diffusion fans 190, from growing. Hence, a period of time required for evaporating the droplets of the sterilizing mist can be reduced. Thus, at least a period of time required for the detoxification process can be reduced, with the result that a period of time required for the sterilizing processing can be reduced.

Further, in the first embodiment, a spray method is used as a method by which the sterilizing unit 140 intermittently sprays the sterilizing mist. Specifically, the flow rate in the peristaltic pump 142b is set lower than the flow rate of the sterilizing liquid drawn into the nozzle 143 by creation of a negative pressure within the nozzle 143 through delivery of the air with the compressor 145a. However, technologies in the present disclosure are not limited thereto. As a method by which the sterilizing unit 140 intermittently sprays the sterilizing mist, for example, the sterilizing mist obtained by conversion into mist with an ultrasonic vibrator may be sprayed by intermittently ejected air. This is effective in that the intermittent spray of the sterilizing mist can be easily controlled. Further, it is also effective in that the particles of the sterilizing mist are less likely to form droplets since the diameter thereof is small.

As described above, the embodiments have been described as exemplification of the technologies in the present disclosure. Accordingly, the accompanying drawings and the detailed description have been provided.

Hence, the constituent elements described in the accompanying drawings and the detailed description may include not only constituent elements required for solving the problems, but also constituent elements not required for solving the problems in order to exemplify the above technologies. Thus, even when those constituent elements not required are described in the accompanying drawings and the detailed description, it should not be immediately recognized that those constituent elements not required should be requirements.

Further, since the embodiments described above are to exemplify the technologies in the present disclosure, various types of modification, replacement, addition, omission and/or the like can be made in the scope of claims or in the scope equivalent thereto.

### [Industrial Applicability]

The present disclosure can be applied to laboratory environment equipment in which worker's hands are inserted into a box-shaped laboratory environment to conduct work.

### [Reference Signs List]

- 100: isolator system
- 110: isolator
- 111: work platform
- 112: front surface plate
- 113: front surface opening
- 117: partition plate
- 118: duct
- 119: display
- 120: centrifuge unit
- 121: centrifuge
- 123: cover
- 130: observation unit
- 131: observation device
- 132: handle
- 133: elevating mechanism
- 140: sterilizing unit
- 141: sterilizing liquid bottle
- 142a: water suction tube
- 142b: peristaltic pump
- 143: nozzle
- 143a: joint
- 144: nozzle
- 145a: compressor
- 145b: intake tube
- 146a: switching valve
- 146b: switching valve
- 147a: switching valve
- 147b: switching valve
- 148: electronic balance
- 149: intake valve
- 150: air conditioning unit
- 150a: intake unit
- 150b: discharge unit
- 151: inlet
- 152: filter
- 153: intake blower
- 154: intake valve
- 155: outlet
- 156: filter
- 157: discharge blower
- 158: discharge valve
- 159: back-flow valve
- 160: control unit
- 170: pass box
- 180: air conditioning unit
- 190: a plurality of diffusion fans
- 190A: first diffusion fan group
- 190B: second diffusion fan group
- 190C: third diffusion fan group
- 191: diffusion fan
- 192: diffusion fan
- 193: diffusion fan
- 194: diffusion fan
- 195: diffusion fan
- 196: diffusion fan
- 197: diffusion fan
- 198: diffusion fan
- 200: incubator

## Claims

1. An isolator comprising:
a box-shaped main body case (110) including a work space (A) isolated from its surroundings;
a spray device (140) configured to spray a sterilizing mist from a nozzle (143) mounted within the main body case (110), the sterilizing mist being obtained by converting a sterilizing liquid into mist; and
a control device (160) configured to control an operation of the spray device (140),
the spray device (140) being configured to spray the sterilizing mist intermittently when spraying, wherein
the isolator further comprises a diffusion fan (191, 192, 193, 194, 195, 196) mounted within the main body case (110) to diffuse the sterilizing mist, **characterized in that**
the control device (160) is configured to cause the diffusion fan (191, 192, 193,194, 195, 196) to intermittently operate in the spray process of the spray device (140), and cause the diffusion fan (191, 192, 193, 194, 195, 196) to continuously operate from an end of the spray process of the spray device (140).

2. The isolator according to claim 1, wherein
the spray device (140) is configured to spray the sterilizing mist, obtained by converting the sterilizing liquid into mist, diagonally from the nozzle (143) mounted on one of left and right sides in an upper portion on one of front and back sides within the main body case (110), toward an other of the left and right sides in a lower portion on an other of the front and back sides.

3. The isolator according to claim 1, wherein
the spray device (140) includes a water suction tube (142a) through which the sterilizing liquid is suctioned, a liquid delivery device (142b) configured to adjust a flow rate of the sterilizing liquid to be suctioned, the nozzle (143) configured to convert the suctioned sterilizing liquid into mist, and a compressor (145a) configured to deliver air into the nozzle (143) and cause the sterilizing liquid to be sprayed from the nozzle (143), and
a flow rate in the liquid delivery device (142b) is set lower than a flow rate of the sterilizing liquid drawn into the nozzle (143) by creation of a negative pressure within the nozzle (143) through delivery of air with the compressor (145a), so that the spray device (140) intermittently sprays the sterilizing mist.

4. The isolator according to any one of claims 1 to 3, wherein
the control device (160) is configured to perform control so that the spray device (140) intermittently performs a spray process of spraying the sterilizing mist, and
the spray device (140) is configured to intermittently spray the sterilizing mist in the spray process.

5. The isolator according to any one of claims 1 to 4, further comprising:
an intake device (150a) configured to take gas into the main body case (110) through an inlet (151) provided in an upper portion of the main body case (110); and
a discharge device (150b) configured to discharge gas from an interior of the main body case (110) through an outlet (155) provided in the upper portion of the main body case (110), wherein a pressure within the main body case (110) can be released from at least one of the inlet (151) and the outlet (155) in the spray process of the spray device (140).

6. The isolator according to claim 1, comprising:
at least two of the diffusion fans (191, 192, 193, 194) whose airflow directions are different from each other, wherein
the control device (160) is configured to cause the at least two of the diffusion fans (193, 194) to operate in an alternate manner in the spray process of the spray device (140).

7. The isolator according to claim 1, further comprising:
an air conditioning device (150) provided outside the main body case (110), the air conditioning device (150) being configured to perform air conditioning in an interior thereof; and
a first diffusion fan (191, 192) mounted within the main body case (110) to diffuse the sterilizing mist, wherein
the control device (160) is configured to control an operation of the first diffusion fan (191, 192) so that the first diffusion fan (191, 192) operates intermittently while the spray device (140) is spraying.

8. The isolator according to claim 7, further comprising, within the main body case (110),
a second diffusion fan (193, 194) whose airflow direction is different from that of the first diffusion fan (191, 192), wherein
the control device (160) is configured to cause each of the first and the second diffusion fans (191, 192, 193, 194) to operate intermittently while the spray device (140) is spraying.

9. The isolator according to claim 8, wherein
the control device (160) is configured to cause the first and the second diffusion fans (191, 192, 193, 194) to operate in an alternate manner while the spray device (140) is spraying.

10. The isolator according to claim 9, wherein
the control device (160) is configured to, while the spray device (140) is spraying,
stop an operation of the second diffusion fan (193, 194) when the first diffusion fan (191, 192) is operating, and
stop an operation of the first diffusion fan (191, 192) when the second diffusion fan (193, 194) is operating.

11. The isolator according to claim 10, wherein
the control device (160) is configured to, while the spray device (140) is spraying, cause each operating time of the first and the second diffusion fans (191, 192, 193, 194) to be shorter than a stop time.

12. The isolator according to any one of claims 7 to 11, wherein
the first diffusion fan (191, 192) is configured to blow air from one of left and right sides to an other side within the main body case (110), and
the second diffusion fan (192, 194) is configured to blow air from an upper side to a lower side within the main body case (110).

13. The isolator according to claim 12, wherein
the spray device (140) is configured to spray the sterilizing mist diagonally from the nozzle (143) mounted on one of left and right sides in an upper portion on one of front and back sides within the main body case (110) toward an other of the left and right sides in a lower portion on an other of the front and back sides, and
the second diffusion fan (193, 194) is configured to blow air from an upper portion on the other of left and right sides to a lower portion on a front surface side within the main body case (110).

14. The isolator according to claim 13, wherein
the spray device (140) includes a water suction tube (142a) through which the sterilizing liquid is suctioned, a liquid delivery device (142b) configured to adjust a flow rate of the sterilizing liquid to be suctioned, the nozzle (143) configured to spray the suctioned sterilizing liquid, and a compressor (145a) configured to deliver air into the nozzle (143) and cause the sterilizing liquid in a mist form to be sprayed from the nozzle (143), and
a flow rate in the liquid delivery device (142b) is set lower than a flow rate of the sterilizing liquid drawn into the nozzle (143) by creation of a negative pressure within the nozzle (143) through delivery of air with the compressor (145a), so that the spray device (140) intermittently sprays the sterilizing mist.

15. The isolator according to claim 14, further comprising:
an intake device (150a) configured to take gas into the main body case (110) through an inlet (151) provided in an upper portion of the main body case (110); and
a discharge device (150b) configured to discharge gas from an interior of the main body case (110) through an outlet (155) provided in the upper portion of the main body case (110), wherein a pressure within the main body case (110) can be released from at least one of the inlet (151) and the outlet (155), while the spray device (140) is spraying.

## Patentansprüche

1. Isolator, der umfasst:
ein kastenförmiges Hauptkörpergehäuse (110), das einen Arbeitsraum (A) enthält, der gegenüber seiner Umgebung isoliert ist;
eine Sprühvorrichtung (140), die so eingerichtet ist, dass sie einen sterilisierenden Nebel über eine Düse (143) versprüht, die im Inneren des Hauptkörpergehäuses (110) installiert ist, wobei der sterilisierende Nebel gewonnen wird, indem eine sterilisierende Flüssigkeit in Nebel umgewandelt wird; und
eine Steuervorrichtung (160), die so eingerichtet ist, dass sie eine Funktion der Sprühvorrichtung (140) steuert,
wobei die Sprühvorrichtung (140) so eingerichtet ist, dass sie beim Sprühen den sterilisierenden Nebel intermittierend versprüht, und
der Isolator des Weiteren ein Streugebläse (191, 192, 193, 194, 195, 196) umfasst, das im Inneren des Hauptkörpergehäuses (110) installiert ist, um den sterilisierenden Nebel zu streuen,
**dadurch gekennzeichnet, dass**
die Steuervorrichtung (160) so eingerichtet ist, dass sie das Streugebläse (191, 192, 193, 194, 195, 196) veranlasst, beim Sprühvorgang der Sprühvorrichtung (140) intermittierend zu arbeiten, und das Streugebläse (191, 192, 193, 194, 195, 196) veranlasst, von einem Ende des Sprühvorgangs der Sprühvorrichtung (140) an kontinuierlich zu arbeiten.

2. Isolator nach Anspruch 1, wobei
die Sprühvorrichtung (140) so eingerichtet ist, dass sie den sterilisierenden Nebel, der gewonnen wird, indem die sterilisierende Flüssigkeit in Nebel umgewandelt wird, über die Düse (143), die an einer linken oder einer rechten Seite in einem oberen Abschnitt einer vorderen oder einer hinteren Seite im Inneren des Hauptkörpergehäuses (110) installiert ist, diagonal auf eine andere von der linken und der rechten Seite in einem unteren Abschnitt an der anderen von der vorderen und der hinteren Seite versprüht.

3. Isolator nach Anspruch 1, wobei
die Sprühvorrichtung (140) ein Wasseransaugrohr (142a), über das die sterilisierende Flüssigkeit angesaugt wird, eine Flüssigkeitszufuhrvorrichtung (142b), die so eingerichtet ist, dass sie eine Strömungsmenge der anzusaugenden sterilisierenden Flüssigkeit reguliert, die Düse (143), die so eingerichtet ist, dass sie die angesaugte sterilisierende Flüssigkeit in Nebel umwandelt, sowie einen Kompressor (145a) enthält, der so eingerichtet ist, dass er Luft in die Düse (143) einleitet und veranlasst, dass die sterilisierende Flüssigkeit über die Düse (143) versprüht wird, und
eine Strömungsmenge in der Flüssigkeitszufuhrvorrichtung (142b) niedriger eingestellt wird als eine Strömungsmenge der sterilisierenden Flüssigkeit, die in die Düse (143) durch Schaffung eines Unterdrucks im Inneren der Düse (143) über Einleitung von Luft mit dem Kompressor (145a) gesaugt wird, so dass die Sprühvorrichtung (140) den sterilisierenden Nebel intermittierend versprüht.

4. Isolator nach einem der Ansprüche 1 bis 3, wobei
die Steuervorrichtung (160) so eingerichtet ist, dass sie Steuerung so durchführt, dass die Sprühvorrichtung (140) einen Sprühvorgang des Versprühens des sterilisierenden Nebels intermittierend durchführt, und
die Sprühvorrichtung (140) so eingerichtet ist, dass sie den sterilisierenden Nebel bei dem Sprühvorgang intermittierend versprüht.

5. Isolator nach einem der Ansprüche 1 bis 4, der des Weiteren umfasst:
eine Einleitvorrichtung (150a), die so eingerichtet ist, dass sie über einen Einlass (151), der sich in einem oberen Abschnitt des Hauptkörpergehäuses (110) befindet, Gas in das Hauptkörpergehäuse (110) einleitet; und
eine Ableitvorrichtung (150b) die so eingerichtet ist, dass sie über einen Auslass (155), der sich in dem oberen Abschnitt des Hauptkörpergehäuses (110) befindet, Gas aus einem Innenraum des Hauptkörpergehäuses (110) ableitet, wobei ein Druck im Inneren des Hauptkörpergehäuses (110) beim Sprühvorgang der Sprühvorrichtung (140) über den Einlass (151) oder/und den Auslass (155) abgelassen werden kann.

6. Isolator nach Anspruch 1, der umfasst:
wenigstens zwei der Streugebläse (191, 192, 193, 194), deren Luftstrom-Richtungen sich voneinander unterscheiden, wobei
die Steuervorrichtung (160) so eingerichtet ist, dass sie die wenigstens zwei der Streugebläse (193, 194) veranlasst, bei dem Sprühvorgang der Sprühvorrichtung (140) abwechselnd zu arbeiten.

7. Isolator nach Anspruch 1, der des Weiteren umfasst:
eine Luftaufbereitungsvorrichtung (150), die sich außerhalb des Hauptkörpergehäuses (110) befindet, wobei die Luftaufbereitungsvorrichtung (150) so eingerichtet ist, dass sie Luftaufbereitung in ihrem Innenraum durchführt; und
ein erstes Streugebläse (191, 192), das im Inneren des Hauptkörpergehäuses (110) installiert ist, um den sterilisierenden Nebel zu streuen, wobei
die Steuervorrichtung (160) so eingerichtet ist, dass sie eine Funktion des ersten Streugebläses (191, 192) so steuert, dass das erste Streugebläse (191, 192) beim Sprühen der Sprühvorrichtung (140) intermittierend arbeitet.

8. Isolator nach Anspruch 7, der des Weiteren im Inneren des Hauptkörpergehäuses (110) ein zweites Streugebläse (193, 194) umfasst, dessen Luftstrom-Richtung sich von der des ersten Streugebläses (191, 192) unterscheidet, wobei
die Steuervorrichtung (160) so eingerichtet ist, dass sie veranlasst, dass das erste und das zweite Streugebläse (191, 192, 193, 194) beim Sprühen der Sprühvorrichtung (140) jeweils intermittierend arbeiten.

9. Isolator nach Anspruch 8, wobei
die Steuervorrichtung (160) so eingerichtet ist, dass sie das erste und das zweite Streugebläse (191, 192, 193, 194) veranlasst, beim Sprühen der Sprühvorrichtung (140) abwechselnd zu arbeiten.

10. Isolator nach Anspruch 9, wobei
die Steuervorrichtung (160) so eingerichtet ist, dass sie, wenn die Sprühvorrichtung (140) sprüht,
eine Funktion des zweiten Streugebläses (193, 194) unterbricht, wenn das erste Streugebläse (191, 192) arbeitet, und
eine Funktion des ersten Streugebläses (191, 192) unterbricht, wenn das zweite Streugebläse (193, 194) arbeitet.

11. Isolator nach Anspruch 10, wobei
die Steuervorrichtung (160) so eingerichtet ist, dass sie beim Sprühen der Sprühvorrichtung (140) bewirkt, dass die Betriebszeit des ersten und des zweiten Streugebläses (191, 192, 193, 194) jeweils kürzer ist als eine Unterbrechungszeit.

12. Isolator nach einem der Ansprüche 7 bis 11, wobei
das erste Streugebläse (191, 192) so eingerichtet ist, dass es Luft von der linken und der rechten Seite auf eine andere Seite im Inneren des Hauptkörpergehäuses (110) bläst, und
das zweite Streugebläse (192, 194) so eingerichtet ist, dass es Luft von einer oberen Seite auf eine untere Seite im Inneren des Hauptkörpergehäuses (110) bläst.

13. Isolator nach Anspruch 12, wobei
die Sprühvorrichtung (140) so eingerichtet ist, dass sie den sterilisierenden Nebel über die Düse (143), die an einer linken oder einer rechten Seite in einem oberen Abschnitt einer vorderen oder einer hinteren Seite im Inneren des Hauptkörpergehäuses (110) installiert ist, diagonal auf eine andere von der linken und der rechten Seite in einem unteren Abschnitt an der anderen von der vorderen und der hinteren Seite versprüht, und
das zweite Streugebläse (193, 194) so eingerichtet ist, dass es Luft von einem oberen Abschnitt an der anderen von der linken und der rechten Seite auf einen unteren Abschnitt an einer Seite der vorderen Fläche im Inneren des Hauptkörpergehäuses (110) bläst.

14. Isolator nach Anspruch 13, wobei
die Sprühvorrichtung (140) ein Wasseransaugrohr (142a), über das die sterilisierende Flüssigkeit angesaugt wird, eine Flüssigkeitszufuhrvorrichtung (142b), die so eingerichtet ist, dass sie eine Strömungsmenge der anzusaugenden sterilisierenden Flüssigkeit reguliert, die Düse (143), die so eingerichtet ist, dass sie die angesaugte sterilisierende Flüssigkeit versprüht, und einen Kompressor (145a) enthält, der so eingerichtet ist, dass er Luft in die Düse (143) einleitet und veranlasst, dass die sterilisierende Flüssigkeit in Form eines Nebels über die Düse (143) versprüht wird, und
eine Strömungsmenge in der Flüssigkeitszufuhrvorrichtung (142b) niedriger eingestellt wird als eine Strömungsmenge der sterilisierenden Flüssigkeit, die in die Düse (143) durch Schaffung eines Unterdrucks im Inneren der Düse (143) über Einleitung von Luft mit dem Kompressor (145a) gesaugt wird, so dass die Sprühvorrichtung (140) den sterilisierenden Nebel intermittierend versprüht.

15. Isolator nach Anspruch 14, der des Weiteren umfasst:
eine Einleitvorrichtung (150a), die so eingerichtet ist, dass sie über einen Einlass (151), der sich in einem oberen Abschnitt des Hauptkörpergehäuses (110) befindet, Gas in das Hauptkörpergehäuse (110) einleitet; und
eine Ableitvorrichtung (150b) die so eingerichtet ist, dass sie über einen Auslass (155), der sich in dem oberen Abschnitt des Hauptkörpergehäuses (110) befindet, Gas aus einem Innenraum des Hauptkörpergehäuses (110) ableitet, wobei ein Druck im Inneren des Hauptkörpergehäuses (110) über den Einlass (151) oder/und den Auslass (155) abgelassen werden kann, wenn die Sprühvorrichtung (140) sprüht.

## Revendications

1. Isolateur comprenant :
un boîtier principal en forme de boîte (110) incluant un espace de travail (A) isolé de son environnement ;
un dispositif de pulvérisation (140) configuré pour pulvériser un brouillard stérilisant depuis une buse (143) montée à l'intérieur du boîtier principal (110), le brouillard stérilisant étant obtenu en convertissant un liquide stérilisant en brouillard ; et
un dispositif de commande (160) configuré pour commander un fonctionnement du dispositif de pulvérisation (140), le dispositif de pulvérisation (140) étant configuré pour pulvériser le brouillard stérilisant de manière intermittente lors de la pulvérisation, dans lequel
l'isolateur comprend en outre un ventilateur de diffusion (191, 192, 193, 194, 195, 196) monté à l'intérieur du boîtier principal (110) pour diffuser le brouillard stérilisant, **caractérisé en ce que**
le dispositif de commande (160) est configuré pour faire fonctionner de manière intermittente le ventilateur de diffusion (191, 192, 193, 194, 195, 196) dans le procédé de pulvérisation du dispositif de pulvérisation (140), et entraîner le fonctionnement de manière continue du ventilateur de diffusion (191, 192, 193, 194, 195, 196) à partir d'une fin du procédé de pulvérisation du dispositif de pulvérisation (140).

2. Isolateur selon la revendication 1, dans lequel
le dispositif de pulvérisation (140) est configuré pour pulvériser le brouillard stérilisant, obtenu par conversion du liquide stérilisant en brouillard, en diagonale depuis la buse (143) montée sur l'un des côtés gauche et droit dans une partie supérieure sur l'une des faces avant et arrière à l'intérieur du boîtier principal (110), vers un autre des côtés gauche et droit dans une partie inférieure sur une autre des faces avant et arrière.

3. Isolateur selon la revendication 1, dans lequel
le dispositif de pulvérisation (140) inclut un tube d'aspiration d'eau (142a) à travers lequel le liquide stérilisant est aspiré, un dispositif de libération de liquide (142b) configuré pour ajuster un débit d'écoulement du liquide stérilisant à aspirer, la buse (143) configurée pour convertir le liquide stérilisant aspiré en brouillard, et un compresseur (145a) configuré pour libérer de l'air dans la buse (143) et entraîner la pulvérisation du liquide stérilisant depuis la buse (143), et
un débit d'écoulement dans le dispositif de libération de liquide (142b) est paramétré pour être inférieur à un débit d'écoulement du liquide stérilisant entraîné dans la buse (143) par la création d'une pression négative à l'intérieur de la buse (143) à travers la libération d'air grâce au compresseur (145a), de sorte que le dispositif de pulvérisation (140) pulvérise de manière intermittente le brouillard stérilisant.

4. Isolateur selon l'une quelconque des revendications 1 à 3, dans lequel
le dispositif de commande (160) est configuré pour exécuter la commande de sorte que le dispositif de pulvérisation (140) exécute de manière intermittente un procédé de pulvérisation pour la pulvérisation du brouillard stérilisant, et
le dispositif de pulvérisation (140) est configuré pour pulvériser de manière intermittente le brouillard stérilisant dans le procédé de pulvérisation.

5. Isolateur selon l'une quelconque des revendications 1 à 4, comprenant en outre :
un dispositif d'admission (150a) configuré pour prélever du gaz dans le boîtier principal (110) à travers un orifice d'admission (151) prévu dans une partie supérieure du boîtier principal (110) ; et
un dispositif de libération (150b) configuré pour libérer le gaz depuis l'intérieur du boîtier principal (110) à travers un orifice de sortie (155) prévu dans la partie supérieure du boîtier principal (110), dans lequel une pression à l'intérieur du boîtier principal (110) peut être relâchée depuis au moins l'un de l'orifice d'admission (151) et de l'orifice de sortie (155) dans le procédé de pulvérisation du dispositif de pulvérisation (140).

6. Isolateur selon la revendication 1, comprenant :
au moins deux des ventilateurs de diffusion (191, 192, 193, 194) dont les sens d'écoulement d'air sont différents l'un de l'autre, dans lequel
le dispositif de commande (160) est configuré pour faire fonctionner le au moins deux des ventilateurs de diffusion (193, 194) d'une manière alternée dans le procédé de pulvérisation du dispositif de pulvérisation (140).

7. Isolateur selon la revendication 1, comprenant en outre :
un dispositif de conditionnement d'air (150) prévu à l'extérieur du boîtier principal (110), le dispositif de conditionnement d'air (150) étant configuré pour exécuter le conditionnement d'air dans un intérieur de celui-ci ; et
un premier ventilateur de diffusion (191, 192) monté à l'intérieur du boîtier principal (110) pour diffuser le brouillard stérilisant, dans lequel
le dispositif de commande (160) est configuré pour commander un fonctionnement du premier ventilateur de diffusion (191, 192) de sorte que le premier ventilateur de diffusion (191, 192) fonctionne de manière intermittente tandis que le dispositif de pulvérisation (140) est en cours de pulvérisation.

8. Isolateur selon la revendication 7, comprenant en outre, à l'intérieur du boîtier principal (110),
un second ventilateur de diffusion (193, 194) dont le sens d'écoulement d'air est différent de celui du premier ventilateur de diffusion (191, 192), dans lequel
le dispositif de commande (160) est configuré pour faire fonctionner de manière intermittente chacun du premier et du second ventilateurs de diffusion (191, 192, 193, 194) tandis que le dispositif de pulvérisation (140) est en cours de pulvérisation.

9. Isolateur selon la revendication 8, dans lequel
le dispositif de commande (160) est configuré pour faire fonctionner le premier et le second ventilateurs de diffusion (191, 192, 193, 194) d'une manière alternée lorsque le dispositif de pulvérisation (140) est en cours de pulvérisation.

10. Isolateur selon la revendication 9, dans lequel
le dispositif de commande (160) est configuré pour, lorsque le dispositif de pulvérisation (140) est en cours de pulvérisation,
stopper un fonctionnement du second ventilateur de diffusion (193, 194) lorsque le premier ventilateur de diffusion (191, 192) fonctionne, et
stopper un fonctionnement du premier ventilateur de diffusion (191, 192) lorsque le second ventilateur de diffusion (193, 194) fonctionne.

11. Isolateur selon la revendication 10, dans lequel
le dispositif de commande (160) est configuré pour, lorsque le dispositif de pulvérisation (140) est en cours de pulvérisation, rendre chacune des durées de fonctionnement du premier et du second ventilateurs de diffusion (191, 192, 193, 194) plus courte qu'un temps d'arrêt.

12. Isolateur selon l'une quelconque des revendications 7 à 11, dans lequel
le premier ventilateur de diffusion (191, 192) est configuré pour souffler de l'air depuis l'un des côtés gauche et droit vers un autre côté à l'intérieur du boîtier principal (110), et
le second ventilateur de diffusion (192, 194) est configuré pour souffler de l'air depuis une face supérieure vers une face inférieure à l'intérieur du boîtier principal (110).

13. Isolateur selon la revendication 12, dans lequel
le dispositif de pulvérisation (140) est configuré pour pulvériser le brouillard stérilisant en diagonale depuis la buse (143) montée sur l'un des côtés gauche et droit dans une partie supérieure sur l'une des faces avant et arrière à l'intérieur du boîtier principal (110) vers un autre des côtés gauche et droit dans une partie inférieure sur une autre des faces avant et arrière, et
le second ventilateur de diffusion (193, 194) est configuré pour souffler de l'air depuis une partie supérieure sur l'autre des côtés gauche et droit vers une partie inférieure sur un côté de surface avant à l'intérieur du boîtier principal (110).

14. Isolateur selon la revendication 13, dans lequel
le dispositif de pulvérisation (140) inclut un tube d'aspiration d'eau (142a) à travers lequel le liquide stérilisant est aspiré, un dispositif de libération de liquide (142b) configuré pour ajuster un débit d'écoulement du liquide stérilisant à aspirer, la buse (143) configurée pour pulvériser le liquide stérilisant aspiré, et
un compresseur (145a) configuré pour libérer de l'air dans la buse (143) et provoquer la pulvérisation du liquide stérilisant sous une forme de brouillard depuis la buse (143), et
un débit d'écoulement dans le dispositif de libération de liquide (142b) est paramétré pour être inférieur à un débit d'écoulement du liquide stérilisant entraîné dans la buse (143) par création d'une pression négative à l'intérieur de la buse (143) à travers la libération d'air avec le compresseur (145a), de sorte que le dispositif de pulvérisation (140) pulvérise de manière intermittente le brouillard stérilisant.

15. Isolateur selon la revendication 14, comprenant en outre :
un dispositif d'admission (150a) configuré pour prélever du gaz dans le boîtier principal (110) à travers un orifice d'admission (151) prévu dans une partie supérieure du boîtier principal (110) ; et
un dispositif de libération (150b) configuré pour libérer du gaz depuis un intérieur du boîtier principal (110) à travers un orifice de sortie (155) prévu dans la partie supérieure du boîtier principal (110), dans lequel une pression à l'intérieur du boîtier principal (110) peut être relâchée depuis au moins l'un de l'orifice d'admission (151) et de l'orifice de sortie (155), lorsque le dispositif de pulvérisation (140) est en cours de pulvérisation.
